# EUROPEAN PATENT APPLICATION

(11) **EP 1 815 868 A2**
(43) Date of publication of application: **08.08.2007**
(21) Application number: 07008861.2
(22) Date of filing: 25.09.2003
(51) Int. Cl.: A61K 47/32, A61K 45/00, A61P 31/04, A61P 43/00

(54) **Drug absorptivity improving agent**

(30) Priority: 26.09.2002 US 414313 P
(62) Divisional of application: 03748580.2
(71) Applicant: Astellas Pharma Inc., Tokyo 103-8411 (JP)
(72) Inventor: Yoshihara, K., Yaizu-shi Shizuoka 425-0072 (JP); Sako, Kazuhiro, Yaizu-shi Shizuoka 425-0072 (JP)
(74) Representative: HOFFMANN EITLE

(57) **Abstract**

The present invention relates to use of one or two or more selected from polyethylene glycol, polyethylene oxide, and polyoxyethylene polypropylene copolymer where the average number of repeating ethylene oxide units per one chain length contained in a molecule is 17 or greater for producing a drug adsorptivity improving agent on the gastrointestinal mucous layers. It became possible to enhance pharmacological effects by applying it to drugs having anti-*H*. *pylori* activity.

## Description

### Technical Field

The present invention relates to use of an ethylene oxide derivative for producing a drug adsorptivity improving agent on the gastrointestinal mucous layers. Specifically, it relates to use of one or two or more selected from polyethylene glycol, polyethylene oxide, and polyoxyethylene polypropylene copolymer where the average number of repeating ethylene oxide units per one chain length is 17 or greater for producing a drug adsorptivity improving agent on the gastrointestinal mucous layers.

### Background Art

Since the existence of *H. pylori* was ascertained from the stomach tissues of gastritis patients, *H. pylori* has been shown to participate in the disease state of stomach and duodenal disorders, such as gastritis and a peptic ulcer. There have been reports of the prevention of recurrence of the ulcer associated with *H*. *pylori,* and the importance of the eradication of *H. pylori* has been recognized. It has further been suggested that there is a cause-effect correlation between the occurrence of stomach cancer and *H. pylori* infection, even in the absence of carcinogens ([non-patent reference 1]).

In the current *H. pylori* eradication method, triple eradication therapy with antibiotics (amoxicillin and clarithromycin) and a proton pump inhibitor (lansoprazole) is a first choice. This is because acid stability of the drug is poor with singular use or concomitant use of two antibiotics due to the fact that the active optimum pH of antibiotics is generally near neutrality, and because the highest eradication rate has thus far been obtained by concomitant use of three drugs. Nevertheless, the eradication rate when 750 mg amoxicillin, 400 mg clarithromycin, and 30 mg lansoprazole are administered twice/day for one week is only 85 to 90%. Furthermore, because of problems including diarrhea, development of resistant bacteria, a large amount of dosage, and reduced compliance that is attributed to the complexity of long-term treatment, a novel *H. pylori* eradication therapy has been desired.

The use of 2-(2-trans-nonenyl)-3-methyl-4(1H)-quinolone derivatives (hereafter, 1-hydroxy-2-(2-transnonenyl)-3-methyl-4(1H)-quinolone is referred to as compound A) alone or in combination with other antibacterial agents and the like, and a reduction in the number of live bacteria *in vivo* when this compound was used alone on *H. pylori* infected animal models (Mongolian gerbils) are recited in [patent reference 1]. Nevertheless, when the use of this compound alone is considered, further augmentation of anti-*H. pylori* activity is necessary and a technology with which compound A is made to effectively act against *H. pylori* has been desired to accomplish this purpose.

*H. pylori* lives in the gastric mucus and surface layer of the gastric mucous membrane epithelial cells and in the spaces in between ([non-patent reference 2]) and therefore, it is necessary to break through the barrier of the mucous layers by some type of means, such as promoting adsorption of the drug on the mucous layers or improving retention in order that the drug will act directly against the *H. pylori*.

On the other hand, ethylene oxide derivatives that are used as bases for formulation, such as polyethylene glycol, polyethylene oxide, and polyoxyethylene polypropylene copolymer, are employed as solubilizing agents, plasticizers, dispersants, or stabilizers. Polyethylene glycol is used, for instance, for stabilization of polypeptides and as a plasticizer of sucralfate-containing compositions, a base for retention in the blood, and the like. For instance, polyethylene oxide is used as a base for controlling dissolution and polyoxyethylene polypropylene copolymer, e.g., Pluronic, is used as a surfactant, solubilizer, emulsifier, dispersant, and the like.

As described above, various ethylene oxide derivatives are used as bases for formulation. However, in connection with technology for augmenting drug activity, there has hitherto been no attempt to use ethylene oxide derivatives in order to improve adsorptivity of a drug on the gastrointestinal mucous layers, and in particular, in order to augment adsorptivity of a drug on the gastrointenstinal mucous layers where *H. pylori* live in order to augment anti-*H*. *pylori* activity.

Consequently, an object of the present invention is to provide a method of use of a specific ethylene oxide derivative for improving adsorptivity of a drug on the gastrointestinal mucous layers.
[Patent reference 1] US No. 6,184,230
[Non-patent reference 1] T. Watanabe et al., Gastroenterol., 115; 642-648 (1998)
[Non-patent reference 2] Y. Akiyama et al., Drug Delivery system, 15-3; 185-192 (2000)

### Disclosure of the Invention

As a result of performing extensive studies under these circumstances, the present inventors found that adsorptivity of the drug of compound A on the gastrointestinal mucous layers is high in the presence of an ethylene oxide derivative. As a result of further studies, the inventors have successfully completed the present invention upon discovering that anti-*H. pylori* activity is particularly augmented when the average number of repeating ethylene oxide units in the ethylene oxide derivatives is greater than 17.

That is, the present invention relates to:
1. use of one or two or more selected from polyethylene glycol, polyethylene oxide, and polyoxyethylene polypropylene copolymer, wherein the average number of repeating ethylene oxide units per one chain length is 17 or greater, for producing a drug adsorptivity improving agent on the gastrointestinal mucous layers;
2. the use of one or two or more selected from polyethylene glycol, polyethylene oxide, and polyoxyethylene polypropylene copolymer, wherein the average number of repeating ethylene oxide units per one chain length is 17 or greater according to the above 1, wherein the drug is an antibacterial agent;
3. the use of one or two or more selected from polyethylene glycol, polyethylene oxide, and polyoxyethylene polypropylene copolymer, wherein the average number of repeating ethylene oxide units per one chain length is 17 or greater according to the above 2, wherein the drug has anti-*H. pylori* activity;
4. use of one or two or more selected from polyethylene glycol, polyethylene oxide, and polyoxyethylene polypropylene copolymer, wherein the average number of repeating ethylene oxide units per one chain length is 17 or greater, for improving adsorptivity of a drug on the gastrointestinal mucous layers;
5. the use of one or two or more selected from polyethylene glycol, polyethylene oxide, and polyoxyethylene polypropylene copolymer, wherein the average number of repeating ethylene oxide units per one chain length is 17 or greater according to the above 4, wherein the drug is an antibacterial agent;
6. the use of one or two or more selected from polyethylene glycol, polyethylene oxide, and polyoxyethylene polypropylene copolymer, wherein the average number of repeating ethylene oxide units per one chain length is 17 or greater according to the above 5, wherein the drug has anti-*H. pylori* activity;
7. a drug adsorptivity improving agent on the gastrointestinal mucous layers, comprising one or two or more selected from polyethylene glycol, polyethylene oxide, and polyoxyethylene polypropylene copolymer, wherein the average number of repeating ethylene oxide units per one chain length is 17 or greater as an active component;
8. the drug adsorptivity improving agent on the gastrointestinal mucous layers according to the above 7, wherein the drug is an antibacterial agent;
9. the drug adsorptivity improving agent on the gastrointestinal mucous layers according to the above 8, wherein the drug has anti-*H*. *pylori* activity;
10. a pharmaceutical composition for improving adsorptivity of a drug on the gastrointestinal mucous layers, comprising at least a drug and one or two or more ethylene oxide derivatives selected from polyethylene glycol, polyethylene oxide, and polyoxyethylene polypropylene copolymer where the average number of repeating ethylene oxide units per one chain length is 17 or greater;
11. the pharmaceutical composition according to the above 10, wherein the drug is an antibacterial agent;
12. the pharmaceutical composition according to the above 11, wherein the drug has anti-*H. pylori* activity;
13. the pharmaceutical composition according to the above 10, wherein the ratios of the components of the composition when the administration form is a liquid are 0.00005% to 50% of the drug and 0.1% to 37.5% of the ethylene oxide derivative and/or 0.1 mg to 1 g of the drug and 2 mg to 1 g of the ethylene oxide derivative per the total composition;
14. the pharmaceutical composition according to the above 10, wherein the ratios of the components of the composition when the administration form is a solid are 0.01% to 95% of the drug and 5% to 99.99% of the ethylene oxide derivative and/or 0.1 mg to 1 g of the drug and 50 mg to 1 g of the ethylene oxide derivative per total composition.

As cited in the present invention, the "gastrointestinal mucus" means an adhesive secretion that is secreted from the gastrointestinal mucous membrane, for instance, the mucus at the stomach walls. The "gastrointestinal mucous layers" refers to layers of the above-mentioned gastrointestinal mucus that are formed on the surface of the gastrointestinal epithelial cells. As also cited in the present invention, the "adsorptivity of a drug on the gastrointestinal mucous layers" means *in vitro* adsorptivity of a drug on the gastrointestinal mucus components, reflecting *in vivo* adsorptivity of the drug. For instance, it is possible to evaluate the adsorptivity by bringing a lipid (oil phase) that is a component of gastrointestinal mucus and a drug suspension (aqueous phase) into contact with one another and then determining the rate of adsorptivity of the drug on the lipid (W. L. Agneta et al., Pharm. Res., 15; 66-71 (1998) was referred to with regard to a mucous layer composition). In the present invention, it appears that when adsorptivity is improved, the "retention" in the gastrointestinal mucous layer is also improved, and in that sense, there are cases where the "retention" is used as a synonym thereof. It is assumed that the ability of a drug to move to the mucous layers also improves with improvement of adsorptivity of the drug on the mucous layers. For convenience, the "improvement of adsorption on the mucous layers" means that, for instance, the rate of adsorption of a drug on the oil phase in the case where an ethylene oxide derivative has been added to the aqueous phase is significantly increased in comparison to the case where the ethylene oxide derivative is not added.

As cited in the present invention, the "ethylene oxide derivative" is a substance containing an ethylene oxide chain in the molecule thereof, and examples include polyethylene glycol, polyethylene oxides, and polyoxyethylene polypropylene copolymer. Of these, there may be mentioned polyethylene glycol 6000 (brand name Macrogol 6000, average relative molecular weight (hereinafter, average molecular weight) of 8000) or polyethylene glycol 20000 (brand name Macrogol 20000, average molecular weight of 20000), polyethylene oxides (average molecular weight of 900,000 or 7,000,000), and polyoxyethylene polypropylene copolymer (e.g., brand name: Pluronic F68, manufactured by Asahi Denka) as preferred examples.

Moreover, as cited in the present invention, the "average number of repeating ethylene oxide units per one chain length" means the number of moles of ethylene oxide units that is added at one position within a molecule as conveniently calculated. Specifically, this is determined by calculating a value obtained by dividing the number of repeating all ethylene oxide units contained in one molecule by the structural number of ethylene oxide chains. The "structural number of ethylene oxide chains" means the number of ethylene oxide chains anywhere in the structure. For example, "the average number of repeating ethylene oxide units per one chain length" can be calculated as follows.

It is realized from the schematic drawing shown in Table 4 that there is one ethylene oxide chain in the case of Macrogol 6000. Consequently, the total number of repeating ethylene oxide units contained in one molecule (n) shown in Table 3 itself becomes "the average number of repeating ethylene oxide units per one chain length (m)". That is, the "average number of repeating ethylene oxide units per one chain length" of Macrogol 400, Macrogol 4000, Macrogol 6000, and Macrogol 20,000 is 8, 72, 188, and 455, respectively. Moreover, in the case of Pluronic, since it has two ethylene oxide chains in its structure (Table 4), the value obtained by dividing the total number of repeating ethylene oxide chains contained in one molecule (n, Table 3) by two is "the average number of repeating ethylene oxide per one chain length." That is, the total number of repeating ethylene oxide units contained in one molecule in L31, L44, L64, P103, P85, and F68 is 3, 20, 27, 29, 54, and 160, respectively; therefore, the "average number of repeating ethylene oxide units per one chain length" becomes 1.5, 10, 13.5, 14.5, 27, and 80, respectively.

Adsorptivity of a drug on the gastrointestinal mucous layers is improved when the "average number of repeating ethylene oxide units per one chain length" is 17 or greater, preferably 27 or greater.

By means of the invention, adsorptivity of compound A and 2-(2-trans-nonenyl)-3-methyl-4(1H) quinolone derivatives on the gastrointestinal mucous layers is improved. Examples of the other drugs include pharmaceutically acceptable antibacterial agents, such as nitroimidazole antibiotics, specifically tinidazole and metronidazole; tetracyclines, specifically tetracycline, minocycline, and doxycycline; penicillins, specifically amoxicillin, ampicillin, talampicillin, bacampicillin, lenampicillin, mezlocillin, and sultamicillin; cephalosporins, specifically cefaclor, cefadroxil, cephalexin, cefpodoxime proxetil, cefixime, cefdinir, ceftibuten, cefotiam hexetil, cefetamet pivoxil, and cefuroxime axetel; penems, specifically, faropenem and ritipenem acoxil; macrolides, specifically erythromycin, oleandomycin, josamycin, midecamycin, rokitamycin, clarithromycin, roxithromycin, and azithromycin; lincomycins (e.g., lincomycin and clindamycin); aminoglycosides, specifically, paromomycin; and quinolones, specifically ofloxacin, levofloxacin, norfloxacin, enoxacin, ciprofloxacin, lomefloxacin, tosufloxacin, fleroxacin, sparfloxacin, temafloxacin, nadifloxacin, grepafloxacin, and pazfloxacin, as well as nitrofurantoin, and the like. Other examples include pharmaceutical compounds that are used to treat diseases associated with stomach acid secretion, such as acid pump inhibitors, specifically omeprazole and lansoprazole; and H2 antagonists, specifically, ranitidine, cimetidine, and famotidine. Further examples include calcium antagonists, specifically, nifedipine, nicardipine hydrochloride, barnidipine hydrochlorid, nitrendipine, and the like. Still further examples include drugs used to treat hyponatremia, specifically 4'-[(2-methyl-1,4,5,6-tetrahydroimidazo [4,5-d] [1]benzazepin-6-yl)carbonyl]-2-phenylbenzanilide hydrochloride; and antigastrin drugs, specifically (R)-1-[2,3-dihydro-1-(2'-methylphenacyl)-2-oxo-5-phenyl-1H-1,4-benzodiazepin-3-yl]-3-(3-methylphenyl)urea, pirenzepine hydrochloride, secretin, and proglumide, and the like. One of these drugs or a combination of two or more of these drugs can be used.

There are no special restrictions to the amount of the drug used in the present invention as long as it is the amount that is effective in terms of treating disease.

It is difficult to unconditionally specify the ratio of each component at the time when they are made into a composition. For instance, when the administration form is a liquid, such as a suspension, there is 0.00005% to 50%, preferably 0.00015% to 0.25%, further preferably 0.0003% to 0.15%, of the drug per entire composition. Moreover, there is 0.1% to 37.5%, preferably 0.1% to 25%, of the ethylene oxide derivative per entire composition. Furthermore, for instance, when the administration form is a solid, such as a powder, it is possible to be 0.01% to 95%, preferably 0.1% to 90%, of the drug per entire composition, and to bring the amount of the ethylene oxide derivative per entire composition to 5% to 99.99%, preferably 10% to 99.9%.

Additionally, for instance, when the administration form is a liquid, it is possible to be 0.00005% to 50%, preferably 0.0001% to 30%, of the drug per entire composition, and to bring the amount of the ethylene oxide derivative per entire composition to 0.1% to 37.5%, preferably 1% to 25%.

There is a fear that sufficient adsorption of the drug will not be obtained in the case where the composition ratio of ethylene oxide is lower than that cited here.

Furthermore, with regard to the amount of each composition that is used, when the administration form is a liquid, for instance, the amount of the drug is brought to 0.1 mg to 1 g, preferably 0.5 mg to 750 mg, and the amount of the ethylene oxide derivative is brought to 2 mg to 1 g, preferably 5 mg to 750 mg.

Alternatively, when the administration form is a solid, for instance, the amount of the drug is brought to 0.1 mg to 1 g, preferably 0.5 mg to 750 mg, and the amount of the ethylene oxide derivative is brought to 50 mg to 1 g, preferably 50 mg to 750 mg.

As with the composition ratio, there is a fear that sufficient adsorption of the drug will not be obtained in the case where the amount used is less than that cited here.

The ethylene oxide derivative of the invention can be made into a pharmaceutical composition for oral use together with the drug and an appropriate excipient and the like that are generally accepted pharmaceutically. There are no special restrictions to the form of the pharmaceutical preparation that the pharmaceutical composition for oral use can take, and a form that can be orally administered, including powders, tablets, capsules, liquids, suspensions, emulsions, and the like can be cited as an example. Formulation can be manufactured by a production method known *per se.*

Pharmaceutical additives, such as excipients, disintegrators, binders, lubricants, fluidizing agents, dispersants, suspending agents, emulsifiers, preservatives, and stabilizers, can be included in the "excipient and the like that are generally accepted pharmaceutically" as cited in the invention.

Examples of excipients are lactose, mannitol, potato starch, wheat starch, rice starch, corn starch, and crystalline cellulose; examples of disintegrators are sodium hydrogen carbonate, and sodium lauryl sulfate; examples of dispersants are crystalline cellulose, dextrin, and citric acid; examples of solubilizing agents are hydroxypropyl methylcellulose, polyoxyethylene-hydrogenated castor oil, cyclodextrins, and polysorbate 80; examples of swelling agents are carboxymethyl cellulose, carboxymethyl cellulose calcium, and croscarmellose sodium; and examples of surfactants are sodium lauryl sulfate and sucrose fatty acid ester. One or two or more can be mixed in appropriate amounts as needed.

The manufacturing method for preparing a pharmaceutical composition for oral use involves, for instance, introducing Macrogol 6000 (polyethylene glycol 6000), a drug (compound A), and an excipient and the like as needed to a pharmaceutically acceptable medium and thoroughly mixing them until they are dissolved and/or suspended. It is possible to select ion-exchanged water, a buffer solution, physiological saline, or the like as the pharmaceutically acceptable medium. Furthermore, the solution and/or suspension can be filled into capsules, such as gelatin capsules, to obtain a capsule form. The method whereby Macrogol 6000, compound A, and a pharmaceutical excipient and the like as needed are granulated by a method known *per se,* such as pulverizing, spray drying, freeze drying, wet granulation, or dry granulation, can be cited as a method of making a powder. Moreover, it is also possible to further blend a pharmaceutical excipient and the like as appropriate and tablet the mixture to obtain a tablet form.

### Brief Description of the Drawings

Figure 1 is a schematic drawing showing drug permeability into the mucous layers.
Figure 2 is a graph showing the effect of the total number of repeating ethylene oxide (POE) units (n) in one molecule on the rate of adsorption of a drug on the oil phase.
Figure 3 is a graph showing the effect of the calculated average number of repeating ethylene oxide (POE) units per one chain length on the rate of adsorption of a drug on the oil phase.
Figure 4 is a graph showing the effect of surface tension on the rate of adsorption of a drug on the oil phase.
Figure 5 is a graph showing the effect of the ethylene oxide (POE) content on the rate of adsorption of a drug on the oil phase.

### Best Mode for Carrying Out the Invention

The following will describe the invention specifically with reference to Examples, but the scope of the invention is not limited by these Examples.

### Example 1

A predetermined amount of compound A was added to ion-exchanged water and a drug suspension was obtained by exposure for 20 minutes to ultrasonic waves (Sono Cleaner, Kaijo Corporation). The suspension was prepared so that each concentration of polyethylene glycol 6000 (Sanyo Chemical Industries, Ltd.; brand name Macrogol 6000) added became 0, 1.5%, 3.5%, 10%, 12%, or 35%.

### Experimental Example 1

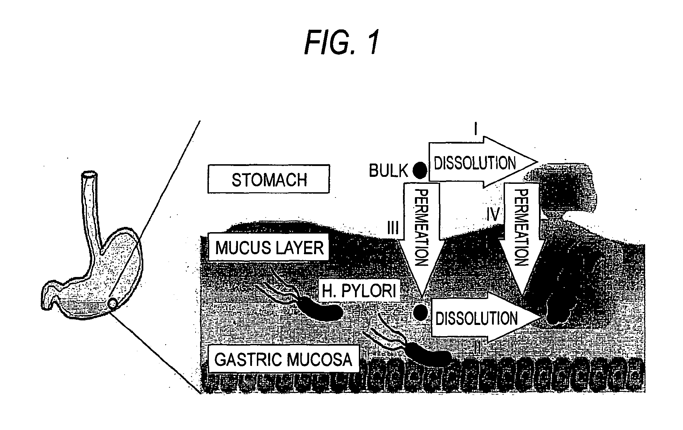

Compound A is a drug that acts directly from the gastric lumen side on the *H. pylori* that lives in the mucous layers and therefore, as shown in Figure 1, the case where the bulk powder that has been administered transfers (IV) after dissolution (I) in the gastric lumen or the case where the bulk powder is dissolved (II) after transferring (III) to the mucous layers will be considered. The effects of Macrogol 6000 on the course of dissolution of compound A were studied. A predetermined amount of compound A was added to ion-exchanged water, a 0.8% mucin (manufactured by Sigma) solution, a 6.2% BSA (manufactured by Sigma) solution, and linoleic acid (manufactured by Sigma), and various drug suspensions were obtained by exposure for 20 minutes to ultrasonic waves (Sono Cleaner, Kaijo Corporation). The procedure in the following was performed as in Example 1 and the following samples were obtained.

### [Samples]

(1) Dispersion of compound A in water (compound A concentration: 530 µg/mL)
(2) Dispersion of compound A in water (compound A concentration: 530 µg/mL) + Macrogol 6000 (3.5%)
(3) Dispersion of compound A in an aqueous mucin solution (0.8%) (compound A concentration: 300 µg/mL)
(4) Dispersion of compound A in an aqueous mucin solution (0.8%) (compound A concentration: 300 µg/mL) + Macrogol 6000 (3.5%)
(5) Dispersion of compound A in an aqueous BSA solution (6.2%)
(6) Dispersion of compound A in an aqueous BSA solution (6.2%) + Macrogol 6000 (3.5%)
(7) Dispersion of compound A in linoleic acid
(8) Dispersion of compound A in linoleic acid + Macrogol 6000 (10%)

### [Method]

The solubility of compound A in water was determined by filtering the liquid after dispersion using a hydrophilic filter (0.45 µm, manufactured by Advantec) and submitting the filtrate to assay by high-performance liquid chromatography (hereinafter, HPLC) (n = 2, sample (1); n = 3, sample (2)).

The solubility of the drug in mucin was determined by filtering the liquid after dispersion using a hydrophilic filter (0.8 µm, manufactured by Advantec) and submitting the filtrate to assay by HPLC (n = 3, samples (3) and (4)).

The liquid after dispersion was filtered with a hydrophilic filter (0.45 µm, manufactured by Advantec), absorbance at 370 nm and 550 nm (corrected for turbidity) was determined under room temperature using an ultraviolet-visible spectrophotometer, and the solubility was determined (n = 3, samples (5) and (6)).

The liquid after dispersion was filtered with a hydrophilic filter (0.45 µm, manufactured by Advantec), absorbance at 366 nm and 550 nm was determined under room temperature using an ultraviolet-visible spectrophotometer, and the solubility was determined (n = 3, samples (7) and (8)).

### [Results and Discussion]

Even though there was not observed a significant increase in the solubility of compound A when Macrogol 6000 was increased up to 3.5% ((2), 0.1 µg/mL) in comparison to the case where Macrogol 6000 was not added ((1), 0.1 µg/mL), augmentation of the in vivo anti-H. *pylori* activity was observed when the formulation where 0.2% of Macrogol 6000 was added to compound A was administered to *H. pylori-*infected animal models (Mongolian gerbils) (Example 4), in comparison to the case where Macrogol 6000 was not used. Therefore, it appears that a main factor in the augmentation of the in vivo anti-*H*. *pylori* activity is not an improved solubility of the drug in water by the Macrogol 6000.

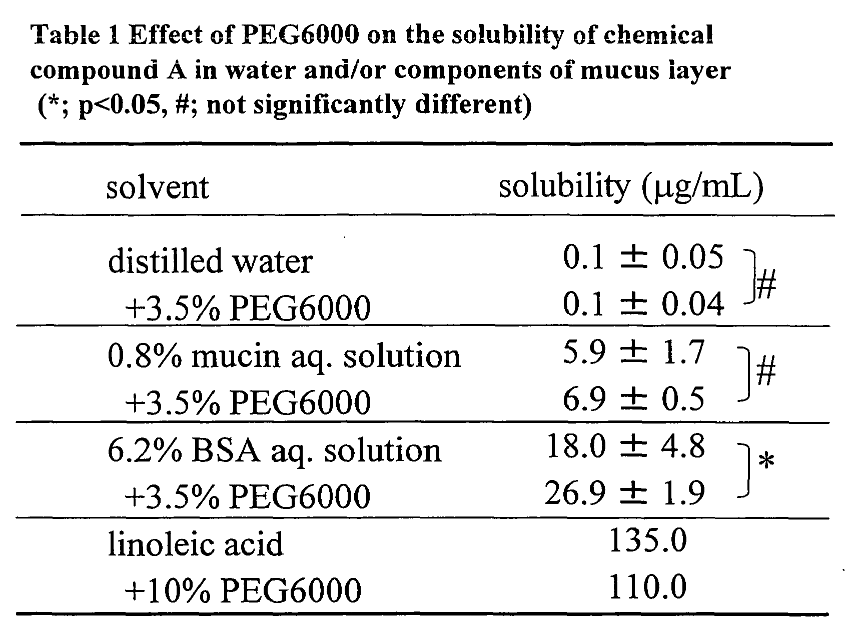

Since the mucous layers in the digestive tract are constituted by water, mucin, proteins and lipids (W. L. Agneta et al., Pharm. Res., 15; 66-71 (1998)), the effects of the addition of Macrogol 6000 on the solubility of compound A in various types of mucus components were investigated (Table 1).

Although the solubility of compound A in an aqueous mucin solution (sample (3), 5.9 µg/mL) was markedly increased as compared to the solubility in water, the increase was only 1.2-fold when Macrogol 6000 was added (sample (4), 6.9 µg/mL). Although the solubility of compound A in BSA solution as a model of a protein in the mucous layers (sample (5), 18.0 µg/mL) was markedly increased as compared to the solubility in water, the increase was only 1.5-fold when Macrogol 6000 was added (sample (6), 26.9 µg/mL). It is reported that the total amount of various lipids in mucus is 37%, and the highest content of the lipids contained is that of linoleic acid at 24% (W. L. Agneta et al., Pharm Res., 15; 66-71 (1998)). Although the solubility of compound A in linoleic acid (sample (7), 135.0 µg/mL) was markedly increased as compared to the solubility in water, an increase was not seen when Macrogol 6000 was added (sample (8), 110.0 µg/mL). Based on the above-mentioned results, it can be assumed that once it has transferred to the mucous layers, compound A is easily dissolved in the mucous layers at or above the bactericidal concentration (concentration that is 10-fold the minimum concentration (0.025 µg/mL) at which an increase in bacteria is inhibited), but the increase in the solubility of compound A in mucous components when Macrogol 6000 is added (1.2-fold to 1.5-fold) is small in comparison to the increase in the adsorption amount of compound A on the oil of the mucus components (2.0-fold, refer to Example 2), and therefore it appears that the increase in the solubility is not a main factor in the augmentation of the in vivo anti-*H. pylori* activity by Macrogol 6000 (refer to Example 4).

According to the above, it appears that the main factor in the augmentation of the in vivo anti-*H. pylori* activity is not an improved solubility of compound A in water or mucus components due to the addition of Macrogol 6000.

### Experimental Example 2

### [Method]

In order to study adsorptivity of a drug from an aqueous phase onto an oil phase (model of mucous layers), an in vitro test system wherein mixing of the oil components in the aqueous phase is prevented was constructed by immobilizing the oil phase with a gelling agent and separating it from the aqueous phase of a drug suspended in a mucin solution. The immobilization of the oil phase was performed by adding 120 mg of an oil gelling agent, which is a natural oil and fat-based fatty acid extracted from castor oil (Johnson Co., Ltd.), to 2 mL of medium chain fatty acid triglyceride (Nihon Oils and Fats Co., Ltd.; brand name: Panaset) to prepare an oil gel in a test tube (inner diameter of 1 cm, Eiken tube No. 5). A solution of 600 µg of compound A suspended in 2 mL of an aqueous 0.8% mucin solution was prepared and brought into contact with the oil phase (n = 6). In the case where Macrogol 6000 was added to the aqueous phase, the concentration was brought to 3.5% (n = 3 to 6). After allowing the solution to stand for two hours, the aqueous phase was recovered and compound A was assayed by HPLC. Furthermore, the surface of the oil phase was washed with methanol and compound A in the recovered solution was assayed by HPLC.

### [Results and Discussion]

As compared to the matter adhered to the surface of the oil phase when the mucin solution alone or the compound A-mucin suspension was brought into contact with the immobilized oil phase and allowed to stand and then the aqueous phase was decanted, an increase in adhered matter was observed when Macrogol 6000 was added. When compound A in the adhered matter was separated and assayed (Table 2), the adsorption amount of the drug on the surface of the oil phase was 259 µg (47% relative to the charged amount) in the case of the compound A-mucin suspension, whereas the amount increased to 506 µg (2.0-fold) when Macrogol 6000 was added, with an average of 93% of the drug that had been added being adsorbed on the surface of the oil phase. Moreover, it was confirmed that the Macrogol 6000 aggregated in the aqueous mucin solution (no drug added), which was attributable to interaction. Based on these facts, it was concluded that when the mucin-Macrogol 6000 aggregate produced by aggregation of Macrogol 6000 and mucin was adsorbed by the oil, the drug was retained in the aggregate and therefore, the increase in the adsorption amount of the drug on the oil phase was observed. On the other hand, because there was no increase in the amount of the drug adsorbed on the oil phase in the case where mucin was not added, regardless of whether or not Macrogol 6000 was added (Table 2), it was suggested that mucin and Macrogol 6000 must both be present for the increase in the adsorption amount of the drug on the oil phase.

**Table 2 Effect of PEG6000 and mucin on the adsorption amount of chemical compound A on the oil-gel phase (*; p < 0.01)**

| water phase | adsorption amount of chemical compound A (µg) | |
|---|---|---|
| | without PEG6000 | with 3.5%PEG6000 |
| mucin (+) | 259 ± 63 | 506 ± 53 * |
| mucin (-) | 249 ± 24 | 173 ± 25 |

| | | |
|---|---|---|
| drug charge; 600µg | | |

Based on the above, it is concluded that in terms of a mechanism of augmenting the in vivo anti-H. *pylori* activity of compound A, the addition of Macrogol 6000 participates little in the improvement of the solubility of compound A in water or in the mucus components (Table 1). Moreover, it can also be assumed that the addition of Macrogol 6000 participates little in the diffusibility of the drug in the mucous layers after dissolution. Consequently, the main factor in augmenting the *in vivo* anti-*H*. *pylori* activity of compound A appears to be that Macrogol 6000 aggregates with mucin and takes up the drug at the time when the aggregate is adsorbed by the lipids (oils) that are mucus components, whereby mucus adsorptivity of compound A is improved.

### Experimental Example 3

### [Method]

The rate of adsorption of compound A on the oil phase was measured by the same method as in Example 2.

### [Results and Discussion]

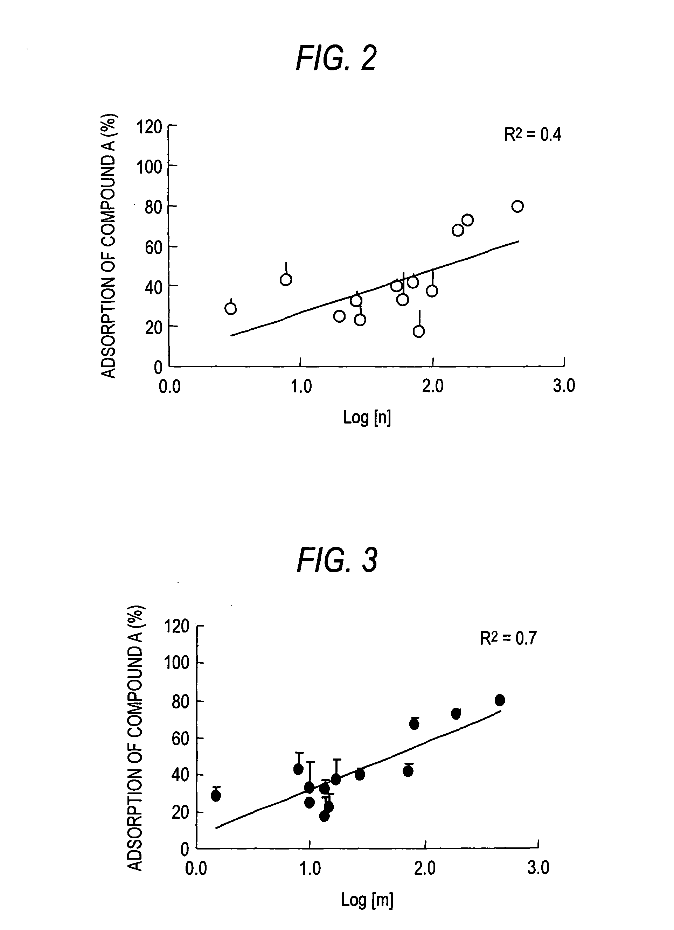

The effect of the number of repeating ethylene oxide (POE) units on the rate of adsorption of a drug on the oil phase (Table 3) was investigated. In contrast to the fact that a significant increase in the rate of adsorption was seen in the case where the number of repeating ethylene oxide units in one molecules (n) was 72 or greater when Macrogol was added and 54 or greater when Pluronic was added, increasing the number of added moles up to 100 had no effect on the rate of adsorption when hydrogenated castor oil (manufactured by Japan Chemicals Co., Ltd., HCO) was added. When the correlation between the rate of adsorption and the number of repeating POE units is analyzed (Figure 2), the correlation coefficient is 0.4, indicating that the correlation between the two is low.

**Table 3 Adsorption of chemical compound A on the oil-gel phase in presence of various excipients contains of polyoxyethylene (POE) units in molecule**

| excipient | n | m | amount(%) *: p<0. O1 |
|---|---|---|---|
| without excipient | 0 | 0 | 19 ± 7 |
| PEG400 | 8 | 8 | 42 ± 9 |
| 4000 | 72 | 72 | 42 ± 4* |
| 6000 | 188 | 188 | 72 ± 3 * |
| 20000 | 455 | 455 | 79 ± 0 * |
| Pluronic L31 | 3 | 1.5 | 28 ± 5 |
| L44 | 20 | 10 | 25 ± 2 |
| L64 | 27 | 13.5 | 32 ± 5 |
| P103 | 29 | 14.5 | 22 ± 7 |
| P85 | 54 | 27 | 40 ± 4 * |
| F68 | 160 | 80 | 67 ± 3 * |
| HCO 60 | 60 | 10 | 33 ± 14 |
| 80 | 80 | 13.3 | 17 ± 10 |
| 100 | 100 | 16.7 | 37 ± 11 |

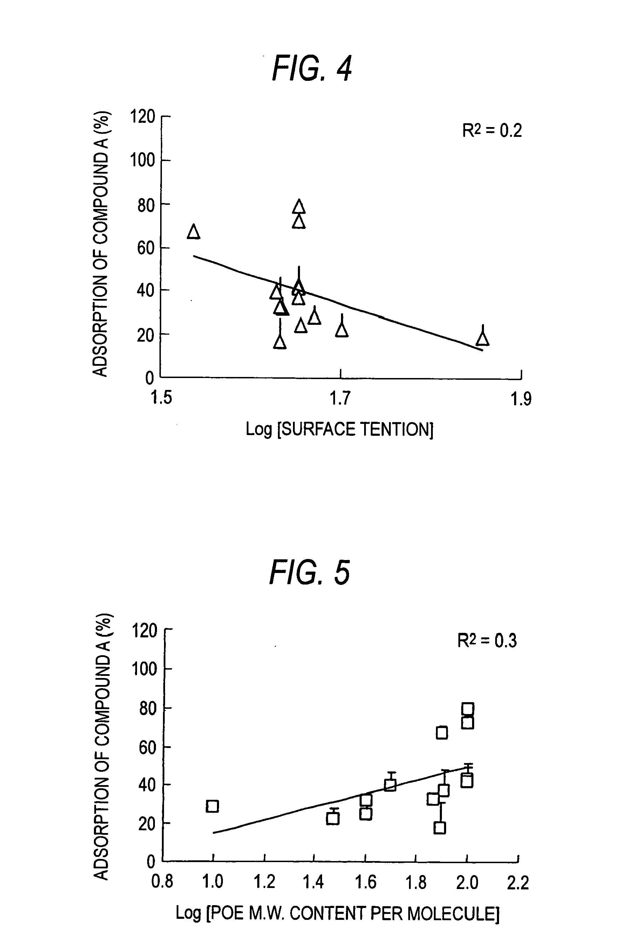

When an average number of repeating POE units per one chain length (m) was determined (Table 3) and its relevancy with the rate of adsorption was analyzed (Figure 3), a high correlation coefficient (0.7) was observed. On the other hand, surface tension showing surface activity or POE content showing hydrophilicity-hydrophobicity balance exhibits a low correlation coefficient (0.2 or 0.3) with the rate of adsorption (Figures 4 and 5). Accordingly, the correlation between the drug adsorption on the oil phase and the average number of repeating POE units per one chain length (m) was suggested and also it was suggested that surface tension and POE content participate little in the rate of drug adsorption.

Based on the above investigation, in the in vitro test method using the immobilized oil phase, increase in the adsorption of the drug on the oil phase was observed at the time when Macrogol and Pluronic were added. It was shown that the adsorption of the drug on the oil phase was not dominated by the interaction between mucin that is a mucus component and the additive but was dominated by the average number of repeating ethylene oxide units per one chain length in the additive.

**Table 4 Chemical structures and schematic images of PEG derivatives**

| *PEG derivative* | *chemical structure* |
|---|---|
| ***PEG*** polyethylene glycol | HO(C₂H₄O)ₙH |
| ***Plurronic surfactant*** polyoxyethylene polyoxypropylene co-polymer | HO(C₂H₄O)ₐ(C₃H₆O)_{c}(C₂H₄O)_{b}H |
| ***HCO surfactant*** polyoxyethylene stearic acid tri-glyceride | 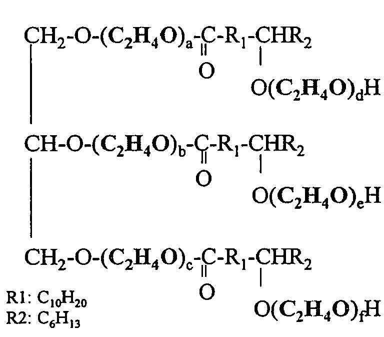 |

### Experiment 4

### [Method]

*In vivo* anti-*H*. *pylori* activity was evaluated with animal experiments using Mongolian gerbil infection models. The sample solutions were drug solutions that had been prepared by suspension of compound A using an 0.5% methylcellulose solution containing 0.2% of Macrogol 6000. The drug was administered twice a day for three days at an administration liquid volume of 20 mL/kg using an oral stomach tube. On the day after the final administration, the stomach was excised by sacrifice and the number of *H*. *pylori* in the stomach were measured. *In vivo* anti-*H*. *pylori* activity was judged from clearance, that is, the ratio of the number of cases in which *H. pylori* was colonized to the number of cases in which the number of bacteria after treatment was below the detection limit.

### [Results and Discussion]

*In vivo* anti-*H*. *pylori* activity was evaluated by animal experiments using Mongolian gerbil infection models (Table 5). The 0.5% methylcellulose suspension (MC sus.) showed clearance of 80% with a dose of 1 mg/kg. When 0.2% of Macrogol 6000 was added to the 0.5% MC sus., clearance was 80% or greater with a dose of 0.1 mg/kg or higher, indicating that there was augmentation (10-fold) of the in vivo anti-*H*. *pylori* activity of compound A. The main factor in augmenting the in vivo anti-*H*. *pylori* activity of compound A appears to be that Macrogol 6000 aggregates with mucin and takes up the drug at the time when the aggregate is adsorbed by the lipids (oils) that are mucus components, and thereby mucus adsorptivity of compound A is improved (refer to Table 2).

It was revealed from the above results in Experiments 1 through 4 that the main factor in the compound A in vivo anti-*H*. *pylori* activity-augmenting mechanism due to the addition of Macrogol 6000 was that the Macrogol 6000 formed an aggregate with the mucin of a mucus component and this aggregate took up the drug when adsorbed on the oil of a mucus component, with the adsorption amount of the drug in vitro on the immobilized oil phase increasing when Macrogol 6000 was added. Since the *in* vivo anti-*H. pylori* activity of compound A was augmented when Macrogol 6000 was added, it was shown that there was a correlation with an increase in the adsorption amount of the drug on the mucus component (oil) in vitro. Furthermore, there was also a correlation between the drug adsorption on the oil and the average number of repeating ethylene oxide units per one chain length, 17 or greater being the average number of repeating ethylene oxide units per one chain length with which there was a significant increase in the drug adsorption in vitro on an immobilized oil phase.

**Table 5 Therapeutic efficacy of chemical compound A against H. pylori infection in Mongolian gerbils**

| **Concentration of PEG6000(%)** | **Clearance rate (Clearance ratio)** | | | | |
|---|---|---|---|---|---|
| | **Dose (mg/kg)** | | | | |
| | ***0*** | ***0.1*** | ***0.3*** | ***1*** | ***3*** |
| **0** | **0%** | **0%** | **20%** | **80%** | **80%** |
| | **(0/5)** | **(0/5)** | **(1/5)** | **(4/5)** | **(4/5)** |
| | **0%** | **80%** | **100%** | **80%** | **100%** |
| **0.2** | **(0/5)** | **(4/5)** | **(4/4)** | **(4/5)** | **(4/4)** |

### Experimental Example 5

### [Method]

The adsorption amount of various drugs in vitro was measured using the method shown in Example 2. A solution of 600 µg of each compound suspended in 2 mL of an aqueous 0.8% mucin solution was prepared and brought into contact with the oil phase (n = 3, 6). When Macrogol 6000 was added into the aqueous phase, it was brought to 3.5% (n = 3, 6). After allowing the solution to stand for 2 hours, the aqueous phase was recovered and the drug content in the aqueous phase was determined by assaying each compound with an ultraviolet-visible spectraphotometer. The compounds that were used were nifedipin, nicardipine hydrochloride, compound B, and compound C. Compound B was (R)-1-[2,3-dihydro-1-(2'-methylphenacyl)-2-oxo-5-phenyl-1H-1,4-benzodiazepin-3-yl]-3-(3-methylphenyl)urea, and compound C was 4'-[(2-methyl-1,4,5,6,-tetrahydroimidazo[4,5-d][1]benzazepin-6-yl)carbonyl]-2-phenylbenzanilide hydrochloride.

Furthermore, the surface of the oil phase was washed with methanol and the drug adsorbed on the oil phase was determined by assaying each compound in the recovered solution with an ultraviolet-visible spectrophotometer.

### [Results and Discussion]

The drug adsorption amount of each compound on the surface of the oil phase when Macrogol 6000 was added increased significantly in comparison to the case where Macrogol 6000 was not added (Table 6). It is considered that this is because the drug was taken up when the aggregate of Macrogol 6000 and mucin was adsorbed on the lipids (oils) that are mucus components.

**Table 6 Effect of PEG6000 on the adsorption amount of various chemical compounds on the oil-gel phase (n=3; mean± SD)**

| chemical compound | adsorption amount of chemical compound( µg) | |
|---|---|---|
| | without PEG6000 | with 3.5%PEG6000 |
| nifedipine | 429 ± 4 | 506 ± 4 * |
| nicardipine | 47 ± 6 | 96 ± 5 * |
| chemical compound B | 269 ± 13 | 411 ± 3 * |
| chemical compound C# | 335 ± 10 | 370 ± 12 * |

| | | |
|---|---|---|
| #; n=6, mean±SD 600 µ g loading *;P<0.01 | | |

### Industrial Applicability

The invention relates to a method of increasing adsorptivity of a drug on the gastrointestinal mucous layers using an ethylene oxide derivative and makes it possible to augment the in vivo anti-*H. pylori* activity of a drug by increasing adsorptivity of the drug on gastrointestinal mucus. Furthermore, the present invention can be applied to singular drug eradication therapy, which has been difficult to accomplish by the current therapies for *H. pylori* eradication, and this will contribute to improvement of compliance.

## Claims

1. A drug adsorptivity improving agent on the gastrointestinal mucous layers, comprising one or two or more selected from polyethylene glycol, polyethylene oxide, and polyoxyethylene polypropylene copolymer, wherein the average number of repeating ethylene oxide units per one chain length is 17 or greater as an active component.

2. The drug adsorptivity improving agent on the gastrointestinal mucous layers according to claim 1, wherein the drug is an antibacterial agent.

3. The drug adsorptivity improving agent on the gastrointestinal mucous layers according to claim 2, wherein the drug has anti-H. *pylori* activity.

4. A pharmaceutical composition for improving adsorptivity of a drug on the gastrointestinal mucous layers, comprising at least a drug and one or two or more ethylene oxide derivatives selected from polyethylene glycol, polyethylene oxide, and polyoxyethylene polypropylene copolymer where the average number of repeating ethylene oxide units per one chain length is 17 or greater.

5. The pharmaceutical composition according to claim 4, wherein the drug is an antibacterial agent.

6. The pharmaceutical composition according to claim 5, wherein the drug has anti-*H*. *pylori* activity.

7. The pharmaceutical composition according to claim 4, wherein the ratios of the components of the composition when the administration form is a liquid are 0.00005% to 50% of the drug and 0.1% to 37.5% of the ethylene oxide derivative and/or 0.1 mg to 1 g of the drug and 2 mg to 1 g of the ethylene oxide derivative per the total composition.

8. The pharmaceutical composition according to claim 4, wherein the ratios of the components of the composition when the administration form is a solid are 0.01% to 95% of the drug and 5% to 99.99% of the ethylene oxide derivative and/or 0.1 mg to 1 g of the drug and 50 mg to 1 g of the ethylene oxide derivative per total composition.
